(19) Europäisches Patentamt
European Patent Office
Office européen des brevets

(11) **EP 3 644 324 A1**

(12) **EUROPEAN PATENT APPLICATION**

(43) Date of publication:
**29.04.2020 Bulletin 2020/18**

(51) Int Cl.:
**G16H 50/20** *(2018.01)* **G16H 50/50** *(2018.01)*

(21) Application number: **18201673.3**

(22) Date of filing: **22.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **AARTS, Ronaldus Maria**
  **5656 AE Eindhoven (NL)**
• **TEN KATE, Warner Rudolph Theophile**
  **5656 AE Eindhoven (NL)**
• **JOHNSON, Mark Thomas**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DETECTING AN ICTAL OF A SUBJECT**

(57) Presented are concepts for detecting an ictal of a subject. One such concept generates an ictal detection threshold based on a current interictal period of the subject. An ictal of the subject may then detected based on an identifier of a potential ictal of the subject and the ictal detection threshold. Adaptation of an ictal detection threshold based on an interictal period of the subject may help to improve the accuracy of ictal detection.

FIG. 2

**Description**

FIELD OF THE INVENTION

This invention relates generally to ictals, and more particularly to detecting an ictal of a subject, such as a person or patient.

BACKGROUND OF THE INVENTION

"Ictal" refers to a physiologic state or event such as a seizure, stroke, or headache. Detecting an ictal of a subject is therefore a concern in many branches of medicine, and of particular concern for subjects having epilepsy or neurological problems.

Systems for detecting are commonly-used in low acuity settings of a hospital or medical care facility to provide an alert in the case of detected ictal of a subject deterioration. One example of a system for detecting an ictal of a subject employs a wrist-worn accelerometer. This enables epileptic seizures (of a certain kind) to be detected. However, such a wrist-worn ictal detector suffers from low specificity (which results in a high number or rate of false or incorrect alerts).

It is a drawback of known systems that they suffer from regular false or incorrect alerts, thereby alerting an ictal event without occurrence of such event. False or incorrect alerts are annoying to a user and can be costly and burdensome for support services.

It may therefore be desirable to provide means or solutions for reducing or preventing the generation of false alerts before, during after an ictal event, or in-between two ictal events..

SUMMARY OF THE INVENTION

The invention aims to at least partly fulfil the aforementioned needs. To this end, the invention provides systems and methods as defined in the independent claims. The dependent claims provide advantageous embodiments.

There is provided a system for detecting an ictal of a subject, the system comprising: a signal interface adapted to obtain a signal comprising an identifier of a potential ictal of the subject; a data acquisition unit adapted to obtain a current interictal period of the subject; a data processing unit configured to generate an ictal detection threshold based on the current interictal period of the subject; an ictal detection component adapted to detect an ictal of the subject based on the identifier of a potential ictal of the subject and the ictal detection threshold; and an output interface configured to output information indicative of the detected ictal of the subject.

By way of explanation, "Pre-ictal" refers to the state immediately before the actual seizure, stroke, or headache. "Post-ictal" refers to the state shortly after the event. "Interictal" refers to the period between seizures, or convulsions, that are characteristic of an epilepsy disorder. For most people with epilepsy, the interictal state corresponds to more than 99% of their life. The interictal period is often used by neurologists when diagnosing epilepsy since an EEG trace will often show small interictal spiking and other abnormalities known by neurologists as subclinical seizures. Interictal EEG discharges are those abnormal waveforms not associated with seizure symptoms. "Peri-ictal" encompasses pre-ictal, ictal and post-ictal.

Proposed is a concept for detecting an ictal of a subject by employing a threshold that is based on a current interictal period of the subject.

By leveraging an understanding that an interictal period tends to extend with duration of the interictal state itself, it is proposed to adapt a detection threshold based on the interictal period (e.g. the time since the last ictal, start time of current interictal period, or duration of current interictal period). In particular, embodiments may increase (i.e. raise) an ictal detection threshold as the duration of the current interictal period increases. Put another way, the ictal detection threshold may be adapted to be proportional to the duration of the current interictal period. For instance, by raising the detection threshold, not only the probability of a false alarm decreases, but the probability of detecting a seizure also decreases. Such odds (i.e. their ratio) may be thought of a kind of metric for the cost/benefit ratio in using the detector. Since the odds change with duration of the interictal period, embodiments may be optimized in terms of keeping the odds constant for example.

Accordingly, by using information about a current interictal period of a monitored subject, an ictal detection threshold may be adapted in accordance with knowledge that occurrence of an ictal may be less likely as the interictal period increases. Proposed concepts may therefore enable the evaluation of whether an identifier of a potential ictal of the subject is actually attributable to an occurrence of an ictal. In this way, embodiments may avoid false alarms that would otherwise be generated as a result of an identifier of a potential ictal of the subject being detected by a conventional ictal detection system.

Further, embodiments may take account of information relating to the previous (i.e. last or most recent) ictal, such as its severity. Severity may, for instance, be measured or indicated by duration and/or strength of activity in sensing signals (e.g. large movements in accelerometer or activity in ECG).

Embodiments may therefore facilitate the identification of a signal (comprising an identifier of a potential ictal) that is meaningful and representative of an ictal occurrence, as distinct from changes caused by physical activity and/or posture changes of the monitored subject for example. This may help to reduce a number of false alarms (i.e. inaccurate or incorrect ictal sensing) and provide more accurate ictal detection. Further, embodiments may be used for evaluation purposes, for example to assess if a subject shows a significant change in ictal occurrence pattern(s).

For example, proposed embodiments may be employed to determine when an identifier (e.g. signal from a sensor) of a potential ictal of the subject is considered reliable and attributable to an ictal occurrence. In this way, improved (e.g. more accurate) detection of an ictal of a subject may be provided by embodiments.

Reliability or accuracy of one or more identifiers of a potential ictal of a subject may therefore be inferred using a single measure, namely current interictal period of the subject.

Information about the current interictal period may be obtained from a control signal provided by a user or control system, or from an output signal of a system identifying the timing of a detected ictal of the subject. This may help to reduce associated cost and/or complexity of an ictal detection system. For instance, embodiments may avoid the need for multiple sensors (and complex signal processing of their respective signals) and may instead simply employ a single interictal period input arrangement.

Embodiments may be based on a proposal to determine an ictal detection threshold based on a current interictal period of the subject. Embodiments may therefore be able to better distinguish between changes in sensed values that represent an actual ictal occurrence, and other changes that may not be caused by the occurrence of an ictal.

Improved (e.g. more accurate) ictal detection may therefore be facilitated by using information relating to the interictal period of the monitored subject.

Proposed embodiments may therefore be of particular relevance to patient monitoring since, for example, it may assist in accurate detection of ictal occurrence for a patient over time. Proposed concepts may also facilitate accurate signalling of improvement or deterioration in the health status of a monitored, yet mobile subject.

In some proposed embodiments, the data acquisition unit may be adapted to obtain information about a current interictal period of the subject based on at least one of: a control signal from a user or control system; and an output signal from the ictal detection component identifying the timing of a detected ictal of the subject. This may facilitate simple and/or cheap provision of information upon which determination of the ictal detection threshold is based.

In proposed embodiments, the data processing unit may be adapted to process the current interictal period in accordance with an interictal model to determine an ictal detection threshold, the interictal model being representative of a relationship between interictal period and a probability of the subject being in an interictal state at a point in time. By way of example, the interictal model may define, for each of a plurality of values of duration of current interictal period, posterior odds of the subject being in an interictal state. The interictal model may be implemented using a look-up table or indexed array, so as to reduce or avoid runtime computations for example.

In an embodiment, the data processing unit may be adapted to determine a value for an ictal detection threshold such that the posterior odds of the subject being in an interictal state given the current interictal period meet a predetermined requirement. By way of example, the predetermined requirement may comprise: the posterior odds remain constant; or the posterior odds decrease with elapsed time. Accordingly, embodiments may be configured so that a ratio between true/correct ictal detections and false/incorrect ictal detections remains constant. Also, simple mathematical functions may be employed, enabling straight-forward and reduced-complexity implementation.

Some embodiments may further comprise a signal generator adapted to generate a signal comprising an identifier of a potential ictal of the subject based on at least one of: physiological data relating to one or more physiological properties of the subject; and activity data relating to activity or movement of the subject. Embodiments may therefore employ existing or conventional ictal sensing systems that are adapted to detect a potential ictal of the subject.

In particular, embodiments may further comprise a first sensor adapted to detect a value of activity or movement of the subject and to generate a signal comprising activity data representative of the detected value of activity or movement. Preferably, the first sensor may comprise at least one of: an accelerometer; a gyroscope; a movement sensor; a weight sensor; a pressure sensor; and a timing device. Further, the sensor may be adapted to be coupled to the subject.

For example, there exist many sensors that can be employed by a system according to an embodiment. Typical sensors include accelerometers, magnetometers, gyroscopes, and air pressure sensors. Accelerometers, for example, can also measure speed or velocity of movement of a subject or an object moved by the subject. Yet another range of sensors consists of microphones and cameras (including infra-red, or even UV and beyond, part of spectrum), to which also belong GPS and location-sensitive IR. Ultra-sound or RF-based sensors, including RFID tagging, provide additional input. Appliances having an own IP-address, known as the internet-of-things, may provide further sensor input signals that can be used by embodiments.

Although the sensor(s) may be mounted in a monitoring environment (e.g. the subject's home), they may also be attached to user utilities (such as a keyring) or put in clothes, in a pocket or bag, or as insole or undergarment, etc. They may also be fabricated to be worn explicitly like a wrist watch or pendant. By way of further example, some embodiments may employ a sensor that is adapted to be coupled to the subject or an object used/manipulated by the subject.

Embodiments may also comprise a second sensor adapted to detect a value of a physiological property of the subject and to generate a signal comprising physiological data representative of the detected value a physiological property. By way of example, the second sensor may comprise at least one of: a blood glucose sensor; a blood pressure sensor; a pulse rate sensor; an electrocardiograph sensor; a skin conductivity (SC) sensor; a temperature sensor; an oxygen saturation (SpO2) sensor; a respiration sensor; a glucose sensor; a muscle tone sensor; and a blood oxygen saturation sensor.

One or more sensors employed by an embodiment may communicate their output signals to an interface of an embodiment via a wired or wireless connection, or a combination thereof. Accordingly, in an embodiment, a sensor may be adapted to be coupled to the subject or the object.

Embodiments may therefore be implemented in conjunction with pre-existing, pre-installed or otherwise separately-provisioned sensors, and the output signals from such sensors may be received and processed in accordance with proposed concepts. Other embodiments may be provided with sensors (e.g. where appropriate sensors are not already available).

A sensor employed by an embodiment may also be adapted to undertake primary processing of the detected values, such a signal filtering, sampling, conditioning, etc., so as to reduce required transmission bandwidth and/or transmission duration for example. Alternatively, a sensor may send raw data.

A sensor arrangement/system may be positioned in a strategic position so that it detects the appropriate value without the subject needing to intentionally or consciously activate/operate the sensor. In this way, a subject may only need to undertake their normal activities. Such strategic positioning may ensure that a value of a property of the subject or object can be automatically and accurately obtained, and this may not require the subject to remember to undertake any special or additional activities in order for a value to be detected by the sensor. This may remove the risk of the subject forgetting to activate a sensor (e.g. by pressing a button), for example.

Non-intrusive monitoring may therefore be realized with relatively simple sensors that provide data on specific properties/parameters of an object or properties of the person (such as movement, speed, and/or distance travelled for example). Such sensors may be simple, small and/or cheap.

Thus, embodiments may employ conventional sensors and/or existing sensor arrangements. Also, embodiments may employ sensors that are considered to be non-intrusive and more easily accepted by the monitored subject. Yet, with the data provided by these sensors, variations in a subject's movement and/or physiological property may be determined and provide information on the subject being monitored.

Such sensors may be employed by, or in conjunction with, embodiments so as to increase accuracy. They may also be used to confirm or qualify readings taken by a primary sensor, so that spurious or unintentional measurements are avoided. For example, signals from a location sensor worn by the monitored subject may be used to confirm if movement readings taken by a movement sensing system are indeed attributable to the monitored subject.

Embodiments may be further adapted to store data in a database adapted to store historical data relating to one or more previously detected ictals and/or previously determined ictal detection thresholds. Previously obtained data and/or determined values may therefore be stored, in a historical database for example, and then used in subsequent calculations. Furthermore, currently determined values may be used to re-calculate or refine a previously determined threshold or detection.

It will be appreciated that all or part of a proposed system may comprise one or more data processing units. For example, the system may be implemented using a single processor which is adapted to undertake data processing in order to detect an ictal of a subject.

The system for detecting an ictal of a subject may be remotely located from the sensor(s), and a signal comprising an identifier of a potential ictal of the subject may be communicated to the system unit via a communication link.

The system may comprise: a server device comprising the signal interface, data acquisition unit, and ictal detection unit; and a client device comprising one or more sensor(s). Dedicated data processing means may therefore be employed for the purpose of detecting an ictal of a subject, thus reducing processing requirements or capabilities of other components or devices of the system.

The system may further comprise a client device, wherein the client device comprises the signal interface, data acquisition unit, data processing unit, ictal detection unit and output interface (e.g. a display system). In other words, a user (such as a care giver) may have an appropriately arranged client device (such as a laptop, tablet computer, mobile phone, PDA, etc.) which processes received data (e.g. identifier of a potential ictal and interictal period) in order to detect an ictal of a monitored subject.

Thus, processing may be hosted at a different location from where the sensing happens. For example, for reasons of power efficiency (e.g. to improve battery lifetime) it might be advantageous to execute only part of the processing at the sensor location, thereby reducing associated costs, processing power, transmission requirements, etc.

Thus, it will be understood that processing capabilities may therefore be distributed throughout the system in different ways according to predetermined constraints and/or availability of processing resources.

Embodiments may also enable some of the processing load to be distributed throughout the system. For example, pre-processing may be undertaken at a sensor system. Alternatively, or additionally, processing could be undertaken at a communication gateway. In some embodiments, processing may be undertaken at a remote gateway or sever, thus relinquishing processing requirements from an end-user or output device. Such distribution of processing and/or hardware may allow for improved maintenance abilities (e.g. by centralising complex or expensive hardware in a preferred location). It may also enable computational load and/or traffic to be designed or located within a networked system according to the processing capabilities available. A preferable approach may be to process sensor signals locally and transmit extracted data for full processing at a remote server.

Embodiments may be further adapted to generate an output signal indicative of the detected ictal of the subject. Embodiments may be adapted to provide an output signal to at least one of: the subject; a medical practitioner; and a caregiver. The output signal may thus be provided to a user or monitoring system for the purpose of indicating if a potential ictal (e.g. as indicated by a signal from a convention ictal sensing system) should be ignored or overlooked for example.

Embodiments may further comprise a user input interface adapted to receive a user input for defining or modifying a current interictal period.

The output interface may be further adapted to generate a control signal for modifying a graphical element based on the detected ictal of the subject. Further, the system may further comprise a display system adapted to display the graphical element in accordance with the control signal. In this way, a user (such as a care giver) may have an appropriately arranged display system that can receive and display information about a detected ictal of the monitored subject, and that user may be remotely located from the subject. Embodiments may therefore enable a user to remotely monitor a subject (e.g. patient) using a portable display device, such as a laptop, tablet computer, mobile phone, PDA, etc.

According to another aspect of the invention, there is provided a method for detecting an ictal of a subject, the method comprising: obtaining a signal comprising an identifier of a potential ictal of the subject; obtaining a current interictal period of the subject; generating an ictal detection threshold based on the current interictal period of the subject; detecting an ictal of the subject based on the identifier of a potential ictal of the subject and the ictal detection threshold; and outputting information indicative of the detected ictal of the subject

According to yet another aspect of the invention, there is provided computer program product for detecting an ictal of a subject, wherein the computer program product comprises a computer-readable storage medium having computer-readable program code embodied therewith, the computer-readable program code configured to perform all of the steps of an embodiment.

A computer system may be provided which comprises: a computer program product according to an embodiment; and one or more processors adapted to perform a method according to an embodiment by execution of the computer-readable program code of said computer program product.

In a further aspect the invention relates to a computer-readable non-transitory storage medium comprising instructions which, when executed by a processing device, execute the steps of the method of controlling a monitoring system display unit according to an embodiment.

These and other aspects of the invention will be apparent from and elucidated with reference to the embodiment(s) described hereinafter.

It will be appreciated by those skilled in the art that two or more of the above-mentioned options, implementations, and/or aspects of the invention may be combined in any way deemed useful.

BRIEF DESCRIPTION OF THE DRAWINGS

Examples in accordance with aspects of the invention will now be described in detail with reference to the accompanying drawings, in which:

Figure 1 is a simplified block diagram of a system for detect in ictal of a subject according to an embodiment;
Figure 2 is a flow diagram of a method for detecting an ictal of a subject according to an embodiment;
Figures 3 is a simplified block diagram of a system for detecting an ictal of a subject according to another embodiment; and
Figure 4 is a simplified block diagram of a computer within which one or more parts of an embodiment may be employed.

DETAILED DESCRIPTION OF THE EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

Proposed is a concept for detecting an ictal of a subject, which may be useful for improving health assessment and monitoring for example. Such subjects may, for instance, include a disabled person, an elderly person, an injured person, a medical patient, etc. Elderly persons can mean persons above 50 years, above 65 years, above 70, or above 80 years old, for example.

Illustrative embodiments may be utilized in many different types of monitoring environments, such as a hospital, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

Embodiments of the present invention are directed toward enabling ictal indications to be identified and potentially dismissed/ignored. Such information may therefore be useful for improved ictal detection accuracy or efficiency, e.g. by avoiding or reducing a number of false alarms.

Embodiments employ the concept of determining an ictal detection threshold based on the duration (i.e. length) of a current interictal period of the subject. Such a current interictal period may be identified by the subject and/or based on a previously detected ictal of the subject for example. By adapting a detection threshold in consideration of a current interictal period, a relationship between interictal period and likelihood of ictal occurrence may be accounted for by embodiments. Warning systems may then be activated/triggered only when an identifier of a potential ictal of the subject is considered to be reliable (e.g. representative of an actual ictal occurrence) in consideration of the detection threshold. An improved ictal detection threshold may therefore be generated based on an interictal period (e.g. identified by user, a sensor and/or previous detections).

By determining an ictal detection threshold, embodiments may enable the identification of signals (e.g. from conventional ictal detection systems) that are significant and/or representative of actual ictal occurrences. This may help to reduce a number of false-positives (i.e. inaccurate or incorrect ictal detections) and provide more accurate ictal detection. Thus, embodiments may be useful for evaluation or monitoring purposes, for example to assess if a subject shows a significant change in a physical health. Reduction of false positives and/or false negatives may therefore be facilitated by proposed embodiments.

Potential ictal occurrences may be detected or inferred from sensor output signals and there already exist systems and methods for such detection or inference. Accordingly, proposed concepts may be used in conjunction with existing ictal detection or monitoring systems/methods. Because many such ictal sensing or detection methods/systems are known and any one or more of these may be employed in conjunction with proposed embodiments, detailed description of such methods/systems is omitted from this description.

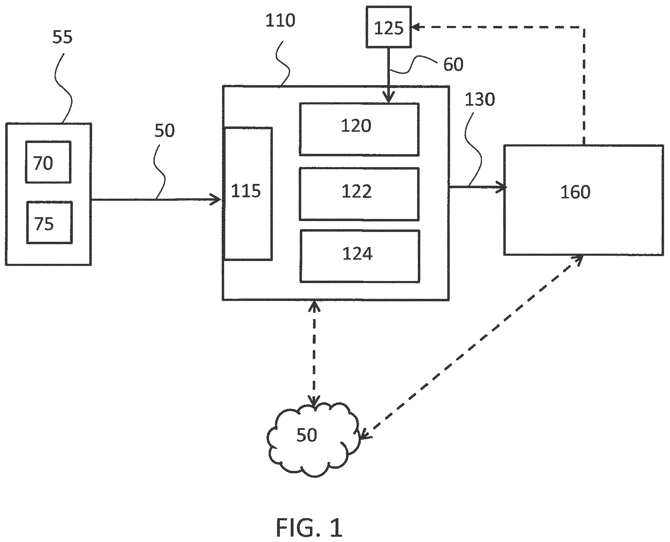

Fig. 1 shows an embodiment of a system according to the invention.

The system includes a signal generator 55 that is adapted to generate a signal 50 comprising an identifier of a potential ictal of a monitored subject. In this example, the signal generator 55 generates such a signal based on: physiological data relating to one or more physiological properties of the subject; and/or activity data relating to activity or movement of the subject.

More specifically, the signal generator 55 comprises a first sensor 70 that is configured to detect a value of activity or movement of the monitored subject. Based on the detected value of activity or movement, the first sensor 70 generates activity data that is representative of the detected value of activity or movement. Here, by way of example only, the first sensor 70 comprises at least one of: an accelerometer; a gyroscope; a movement sensor; a weight sensor; a pressure sensor; and a timing device.

The signal generator 55 also comprises a second sensor 75 that is configured to detect a value of a physiological property of the monitored subject. Based on the detected value of the physiological property, the second sensor 75 generates physiological data representative of the detected physiological property. By way of example, the second sensor 75 comprises at least one of: a blood glucose sensor; a blood pressure sensor; a pulse rate sensor; an electrocardiograph sensor; a respiration sensor; and a blood oxygen saturation sensor.

In this example, the first 70 and second 75 sensors are integrated into a portable device that is coupled to, carried or worn by the monitored subject. In this way, the monitored subject need only undertake his/her normal activities when being monitored and may not even be aware that he/she is operating the signal generator 55 and being monitored. Such configuration of the signal generator 55 may enable movement, activity and/or properties of the subject to be detected without requiring the subject to remember to undertake any special or additional activities. For example, it can remove the need for a subject to perform a specific additional action (e.g. pressing a button) in order to generate a signal 50 comprising an identifier of a potential ictal.

The signal generator 55 is adapted to output a signal 50 that includes an identifier of a potential ictal of a monitored subject. Of course, many more sensors may be employed so as to provide signals indicative of potential ictals of the monitored subject. Such additional signals may also be used to confirm or qualify values detected by the sensors 70,75, so that spurious or unintentional measurements are avoided. For example, signals from a location sensor worn by the monitored subject may be used to confirm if values detected by the first sensor 70 are indeed attributable to the monitored subject walking, for example.

The signal generator 55 communicates its output signals 50 via a wired or wireless connection. By way of example, the wireless connection may comprise a short-to-medium-range communication link. For the avoidance of doubt, short-to-medium-range communication link may be taken to mean a short-range or medium-range communication link having a range of up to around one hundred (100) meters. In short-range communication links designed for very short communication distances, signals typically travel from a few centimetres to several meters, whereas, in medium-range communication links designed for short to medium communication distances, signals typically travel up to one hundred (10)0 meters. Examples of short-range wireless communication links are ANT+, Bluetooth, Bluetooth low energy, IEEE 802.15.4, ISA100a, Infrared (IrDA), , Near Field Communication (NFC), RFID, 6LoWPAN, UWB, Wireless HART, Wireless HD, Wireless USB, ZigBee. Examples of medium-range communication links include Wi-Fi, ISM Band, Z-Wave. Here, the output signals are not encrypted for communication via the wired or wireless connection in a secured manner. However, it will be appreciated that, in other embodiment, one or more encryption techniques and/or one or more secure communication links may be employed for the communication of signals in the system.

The system further comprises a system 110 for detecting an ictal of a monitored subject. The system 110 has a signal interface 115 adapted to receive the signals 50 from the signal generator 55. In this way, the signal interface 115 is adapted to receive a signal (50) comprising an identifier of a potential ictal of the monitored subject.

The system 110 also comprises a data acquisition unit 120 adapted to obtain a current interictal period of the subject. Here, the data acquisition unit 120 receives a control signal 60 from a control system 125 (that may be operated by the monitored subject or his/her carer). However, as depicted by the dashed line, the control signal 60 from the control system 125 may also be based on an output signal from the system which identifies the timing of a detected ictal of the subject.

The obtained information about the current interictal period of the subject is provided to a data processing unit 122 of the system 110. The data processing unit 122 is adapted to generate an ictal detection threshold based on the current interictal period of the subject. For this purpose, data processing unit 122 of the system 110 may communicate with one or more data processing resources available in the internet or "cloud" 50. Such data processing resources may undertake part or all of the processing required to determine an ictal detection threshold.

In this example, the data processing unit 122 processes the current interictal period in accordance with an interictal model to determine an ictal detection threshold. The interictal model is representative of a relationship between interictal period and a probability of the subject being in an interictal state at a point in time. More specifically, the data processing unit 122 is adapted to determine a value for an ictal detection threshold such that the posterior odds of the subject being in an interictal state given the current interictal period meet a predetermined requirement (such as: the posterior odds remain constant; or the posterior odds decrease with elapsed time).

The determined ictal detection threshold is provided to an ictal detection component 124 of the system 110 along with the identifier of a potential ictal of the subject (obtained via the signal interface 115). The ictal detection component 124 is adapted to detect an ictal of the subject based on the identifier of a potential ictal of the subject and the ictal detection threshold.

Again, for this purpose, the ictal detection component 124 may communicate with one or more data processing resources available in the internet or "cloud" 50. Such data processing resources may undertake part or all of the processing required to detect an ictal of the subject.

Thus, it will be appreciated that the embodiment may employ distributed processing principles.

The system 110 is further adapted to generate an output signal 130 representative of the detected ictal of the monitored subject. In other words, after detect an ictal of the subject (either with or without communicating with data processing resources via the internet or "cloud"), an output signal 130 representative of or determined measure of reliability is generated.

The system further comprises an output interface 160, for instance a graphical user interface (GUI) for providing information to one or more users. The output signal 130 is provided to the GUI 160 via wired or wireless connection. By way of example, the wireless connection may comprise a short-to-medium-range communication link. As indicated in Figure 1, the output signal 130 is provided to the GUI 160 from the data processing unit 110. However, where the system, has made use of data processing resources via the internet or cloud 50), an output signal may be made available to the GUI 160 via the internet or cloud 50.

Based on the output signal 130, the GUI 160 is adapted to communicate information by displaying one or more graphical elements in a display area of the GUI 160. In this way, the system may communicate information about a detected ictal that may be useful for indicating a subject's physical state. For example, the GUI 160 may be used to display graphical elements to a medical practitioner, a caregiver, a family member or close relative. Alternatively, or in addition, the GUI 160 may be adapted to display graphical elements to the monitored subject.

From the above description of the embodiments of Figure 1, it will be understood that there is proposed a system for identifying whether an obtained identifier of a potential ictal of the subject is erroneous or attributable to an actual ictal occurrence.

The system can be considered to comprise three main sub-systems/functions: (i) The first is a sensor of a potential ictal - this may, for example, comprise a sensors arrangement that can detect one or more properties of a subject; (ii) The second implements a function for determining an ictal detection threshold based on a current interictal period of the monitored subject; and (iii) The third implements an algorithm which detects an ictal by analysing the sensed potential ictal in conjunction with the ictal detection threshold.

From the above description, it will be appreciated that there is proposed a concept for leveraging knowledge that interictal period between ictal tends to extend with duration of that interictal state itself. To account for this, the detection threshold of an ictal may be adapted based on the interictal period (i.e. time since the last ictal). For instance, the threshold may be increased as the interictal period increases, such that a ratio between true/correct ictal indications and false/incorrect ictal indications is kept constant for example. This may address the issue of false alarms being generated with increased sensitivity or low specificity of an ictal sensor. Proposed embodiments may therefore enable a user to detect ictal occurrence more accurately and/or reliably.

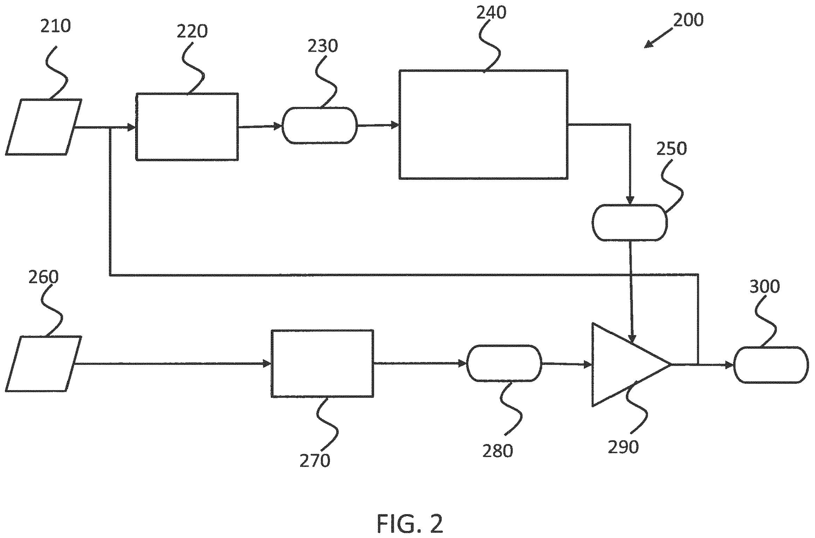

Referring now to Figure 2, there is depicted a simplified flow diagram of a method 200 that may be employed by an embodiment. The method employs accelerometer data (from an accelerometer-based ictal detector) and adapts an ictal detection threshold based on a current interictal period for the monitored subject.

A user or control system 210 provides a signal identifying the timing to of the last ictal of the monitored subject. Based on this indication of most recent ictal, the method determined (in step 220) the current interictal period 230 (i.e. $t-t_0$) of the monitored subject. The interictal period 230 is provided to a process 240 that employs an interictal model to determine an ictal detection threshold $\theta$ 250 based on the current interictal period 230.

Here, the threshold comprises a likelihood value. However, it will be appreciated that the threshold $\theta$ 250 can be other suitable values for testing sensed events, such as the duration and/or intensity of a tremor.

A sensor 260 provides a signal comprising an identifier of a potential ictal of the subject. Here, the sensor 260 comprises a conventional (accelerometer-based) ictal detector. The signal is provided to a process 270 that computes the likelihood ratio 280 that the given event (signal set) is due to an epileptic seizure: $\Lambda$(event) = P(event|seizure) / P(event|NOT seizure).

The computed likelihood ratio 280 is compared with the detection threshold 250 in step 290 to determine if the detected event (e.g. signal set from the sensor 260) is due to an ictal occurrence (e.g. epileptic seizure). Based on the result of the comparison in step 290, an output signal 300 indicative of the detected ictal is generated and output. The output signal 300 may, for example, comprise information identifying the timing and type of the detected ictal. Accordingly, it will be appreciated that the output signal 300 can be provided to step 220 to indicate the timing of the most recent ictal. In this way, the interictal period may be automatically updated upon the detection of a new ictal.

By way of further explanation of the embodiment of Figure 2, and more particularly the interictal model, an example interictal model is described in Suffczynski et al., Dynamics of Epileptic Phenomena Determined From Statistics of Ictal Transitions, IEEE TRANSACTIONS ON BIOMEDICAL ENGINEERING, VOL. 53, NO. 3, pp.524- 532, MARCH 2006. The model consists of a two state system: ictal (= epileptic seizure) and interictal state. The probability to be staying in a state is modeled by a gamma distribution (i.e. one model per state):

$$y = Cx^{\alpha-1}e^{\frac{-x}{\beta}}$$

$$(\text{i})$$

wherein for (some of) the interictal state it is found that $\alpha<1$.

We can match this probability to a survival function S(t) [2]:

$$\lambda(t) = \frac{1}{S(t)} \lim_{\Delta t \downarrow 0} \frac{S(t) - S(t + \Delta t)}{\Delta t} = -\frac{d \log S(t)}{dt}$$

$$(\text{ii})$$

solving (ii) leads to:

$$S(t) = \exp(-\Lambda(t)) \tag{iv}$$

where $\Lambda$ is given by:

$$\Lambda(t) = \int_0^t \lambda(s)\,ds \tag{iii}$$

S(t) expresses (predicts) the probability to be in the interictal state at time t. It is therefore the output of the process 240. Note that the lambda's in the formulas above denote incident rate, while $\Lambda$ used in the earlier equations is used to denote the likelihood ratio.

By matching the gamma distribution (i) to the survival function S(t), we find:

$$\lambda(t) = \lambda_0 + (1-\alpha)/t \tag{iv}$$

For $\alpha=1$, S(t) is a Poisson process ($\lambda(t)$ is constant).

With $\alpha<1$, $\lambda$ (rate of seizures happening) decreases with time, and hence the probability S(t) increasing to stay in the interictal state.

As explained above, for the seizure detection, the embodiments is adapted to test whether the likelihood ratio $\Lambda$(event) = P(event|seizure) / P(event|NOT seizure) passes the threshold $\theta$ 250. The time since last seizure is not a characteristic of the event itself and hence not changing $\Lambda$(event).

However, since the incident rate of the seizures is changing, the odds do change. It is known from statistics that the likelihood ratio equals the odds ratio: it modifies the prior odds into the posterior odds:

$$O(\text{Seizure}|\text{event}) = \Lambda(\text{event}) \cdot O(\text{Seizure}) \tag{v}$$

wherein O(Seizure) is the prior odds = P(seizure) / P(not-a-seizure) and O(Seizure|event) is the posterior odds, the odds on a seizure given the event.

This relationship is used to modify the threshold $\theta$ 250.

By way of example, a service provider (e.g. carer or call center) can agree with a user on a service level that corresponds to a certain ratio of true ictals against false ictals. This ratio is given by the posterior odds, which, as detailed above, equals the product of the likelihood ratio $\Lambda$(event) and prior odds. At initiation, the threshold $\theta$ can be set to $\theta_0$ such that the agreed posterior odds equals $\theta_0$ times the prior odds: $\theta_0$ x O(Seizure).

Now, with time in the interictal state, i.e. time $(t-t_0)$ since last seizure, the threshold $\theta(t-t_0)$ may be adapted such that the posterior odds stay constant:

$$\theta(t-t_0) \cdot O(\text{Seizure}) = \text{constant} \tag{vi}$$

From the above, the prior odds decrease with the decreasing incident rate of seizures:

$$O(\text{Seizure}) = P(\text{seizure}) / P(\text{not-a-seizure}) = \lambda_s(t) / \lambda_{ns} \tag{vii}$$

where $\lambda_{ns}$ is the incident rate of non-seizures, and $\lambda_s(t)$ that of seizures. The latter is given above by the Interactal Model: $\lambda_s(t) = \lambda_0 + (1-\alpha)/t$

Hence, we get (in this embodiment):

$$\theta(t-t_0) = \text{constant} \cdot \lambda_{ns} / \lambda_s(t-t_0) = \text{constant} \cdot \lambda_{ns} / [\lambda_0 + (1-\alpha)/(t-t_0)] \tag{viii}$$

For small t this simplifies to:

$$\theta(t\text{-}t_0) = \text{constant} \; . \; [\lambda_{ns} \, / \, (1\text{-}\alpha)] \; . \; (t\text{-}t_0) \qquad\qquad (ix)$$

i.e. the threshold increases linearly with time.

For large t, the threshold saturates at: constant. $\lambda_{ns} \, / \, \lambda_0$

It will therefore be appreciated that the above-described embodiment adapted the threshold to keep the posterior odds constants. In another embodiment, the posterior odds could also decrease (as the prior odds is doing), but at a less quick pace. Other embodiments are also conceivable.

In a further refinement, next to a reset of $t_0$, the threshold can be lowered again by other mechanisms. For example, in case a potential seizure is witnessed, but its likelihood is below the current threshold, the threshold is lowered with duration of this potential seizure. In a similar way, a (dense) sequence of potential seizures may have the effect to lower the threshold.

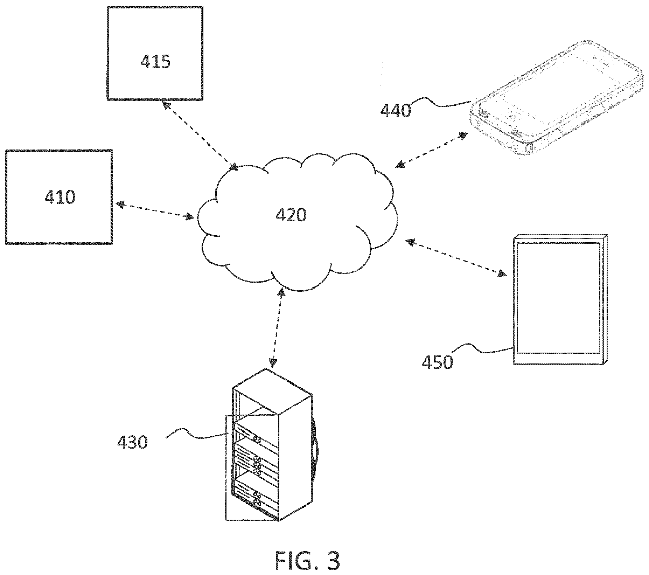

Referring now to Fig. 3, there is depicted another embodiment of a system according to the invention comprising an accelerometer arrangement 410 adapted to detect movement of the subject. Here, the accelerometer arrangement 410 comprises a high-resolution tri-axis accelerometer arrangement 410 adapted to be integrated into a wristwatch that is worn by the monitored subject. The accelerometer arrangement 410 is adapted to output one or more signals which are representative of the detected value(s) of a subject's movement.

Although this embodiment has been described as employing a portable and wearable sensor arrangement, it will be understood that, in alternative embodiments, the movement of the subject may be detected using one or more sensors strategically positioned within a monitoring environment.

The sensor 10 that measures the subject's movement might, or might not be, incorporated in the same device as a vital sign sensor 415. Movement sensor 410 might even be the same sensor as vital signs sensor 415. For example, a sensor integrated into a wristwatch, might both be used to measure movement and to measure pulse rate. On the other hand, the movement sensor 410 might be completely separated from vital sign sensor 415. For example, when the movement is measured with a device worn on the subject's wrist, physiological data may come from a SpO2 sensor on the subject's finger.

The accelerometer arrangement 410 and the vital sign sensor 415 each communicate their respective output signals via the internet 420 (using a wired or wireless connection for example) to a remotely located data processing system 430 (such as server) for detecting an ictal.

The data processing system 430 is adapted to receive the one or more output signals from the accelerometer arrangement 410 (e.g. as activity data) and the vital sign sensor 415 (e.g. physiological data). The system may also adapted to obtain further physiological data relating to one or more physical or physiological attributes of the monitored subject (e.g. from a local or remote database and/or via a user input interface).

The data processing system 430 processes the activity data and physiological data in accordance with a method according to a proposed embodiment to determine an identifier of a potential ictal of the subject.

The data processing system 430 also generates an ictal detection threshold based on a current interictal period of the subject. More specifically, the data processing system 430 processes the current interictal period in accordance with an interictal model to determine an ictal detection threshold.

Based on the identifier of a potential ictal of the subject and the ictal detection threshold, the data processing system 430 detects an ictal of the subject.

The data processing system 430 is further adapted to generate output signals representative of a detected ictal of the monitored subject. Thus, the data processing 430 provides a centrally accessible processing resource that can receive information from the accelerometer arrangement 410 and the vital signs sensor 415 and run one or more algorithms to detect the occurrence of an ictal. Information relating to the detected ictal can be stored by the data processing system 430 (for example, in a database) and provided to other components of the system.

For the purpose of receiving information about a detected ictal of the monitored subject from the data processing system 430, and thus to enable the subject to be monitored accurately and/or in reliably, the system further comprises first 440 and second 450 mobile computing devices.

Here, the first mobile computing device 440 is a mobile telephone device (such as a smartphone) with a display for displaying graphical elements representative of a subject's physical or mental well-being. The second mobile computing device 450 is a mobile computer such as a Laptop or Tablet computer with a display for displaying graphical elements representative of a subject's health.

The data processing system 430 is adapted to communicate output signals to the first 440 and second 450 mobile computing devices via the internet 420 (using a wired or wireless connection for example). This may be undertaken in response to receiving a request from the first 440 or second 450 mobile computing devices.

Based on the received output signals, the first 440 and second 450 mobile computing devices are adapted to display one or more graphical elements in a display area provided by their respective display. For this purpose, the first 440 and second 450 mobile computing devices each comprise a software application for processing, decrypting and/or interpreting received output signals in order to determine how to display graphical elements. Thus, the first 440 and second 450 mobile computing devices each comprise a processing arrangement adapted to receive information indicative of the detected ictal of the subject, and to generate a display control signal for modifying at least one of the size, shape, position, orientation, pulsation or colour of the graphical element based on the information indicative of the detected ictal of the subject.

The system can therefore communicate information about a detected ictal of the subject to users of the first 440 and second 450 mobile computing devices. For example, each of the first 440 and second 450 mobile computing devices may be used to display graphical elements to a medical practitioner, a caregiver, a family member or close relative.

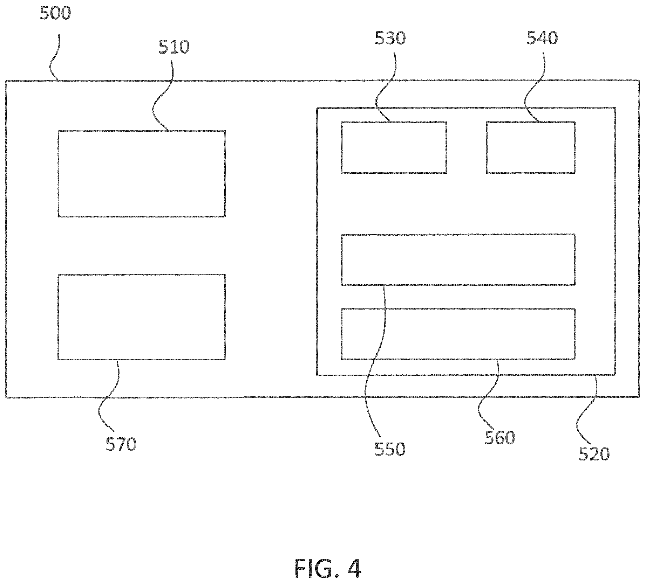

Implementations of the system of Figure 4 may vary between: (i) a situation where the data processing system 430 communicates display-ready data, which may for example comprise display data including graphical elements (e.g. in JPEG or other image formats) that are simply displayed to a user of a mobile computing device using conventional image or webpage display (which can be web based browser etc.); to (ii) a situation where the data processing system 430 communicates raw data set information that the receiving mobile computing device then processes to detect an ictal, and then displays graphical elements based on the detected ictal (for example, using local software running on the mobile computing device). Of course, in other implementations, the processing may be shared between the data processing system 430 and a receiving mobile computing device such that part of the data generated at data processing system 430 is sent to the mobile computing device for further processing by local dedicated software of the mobile computing device. Embodiments may therefore employ server-side processing, client-side processing, or any combination thereof.

Further, where the data processing system 430 does not 'push' information (e.g. output signals), but rather communicates information in response to receiving a request, the user of a device making such a request may be required to confirm or authenticate their identity and/or security credentials in order for the information to be communicated.

It is also noted that, although it has been described above that embodiments need not employ additional/supplementary sensors, some embodiments may further comprise a sensor adapted to detect a value of a property of the monitored subject. Such a supplementary sensor arrangement may help to improve the accuracy of activity data or physiological data for example. Supplementary sensor readings may, for instance, qualify or refine data analysis undertaken.

The sensors may also be adapted to undertake primary processing of the detected values, such a signal filtering, sampling, conditioning, etc., so as to reduce a required transmission bandwidth and/or transmission duration for example.

Non-intrusive monitoring may therefore be realized with relatively simple sensors that provide data on specific properties of the subject (such as movement, for example). Also, potential ictals of the subject may be detected with sensors that are cheap and widely employed. Thus, embodiments may employ sensors that are considered to be non-intrusive and more easily accepted by the monitored subject. Yet, with the data provided by these sensors, combined with information about a current interictal period of a subject, an ictal of the subject may be accurately detected. Thus, some embodiments of the invention may employ conventional ictal sensors and/or existing ictal sensing arrangements. Also, embodiments may employ sensors that are considered to be non-intrusive and more easily accepted by the monitored subject.

Figure 4 illustrates an example of a computer 500 within which one or more parts of an embodiment may be employed. Various operations discussed above may utilize the capabilities of the computer 500. For example, one or more parts of a monitoring system adapted to monitor a subject may be incorporated in any element, module, application, and/or component discussed herein.

The computer 500 includes, but is not limited to, PCs, workstations, laptops, PDAs, palm devices, servers, storages, and the like. Generally, in terms of hardware architecture, the computer 500 may include one or more processors 510, memory 520, and one or more I/O devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 540, source code 530, and one or more applications 560 in accordance with exemplary embodiments. As illustrated, the application 560 comprises numerous functional components for implementing the features and operations of the exemplary embodiments. The application 560 of the computer 500 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. It is contemplated by the inventors that the application 560 for implementing exemplary embodiments may be applicable on all commercially available operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, php. Python, ASP scripts, FORTRAN, COBOL, Perl, Java, ADA, .NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at startup, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, optimized for embedded implementation, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

From the above description, it will be appreciated that embodiments may therefore be useful for monitoring of individuals susceptible to ictal occurrence so to support independent living. Embodiments be used both for real-time monitoring and alerts, as well as to detect when other sensing/detection approaches are/aren't reliable and/or when ictal occurrences deviate from usual or expected patterns or trends.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand that various embodiments with various modifications are contemplated.

## Claims

1. A system for detecting an ictal of a subject, the system comprising:

   a signal interface (115) adapted to obtain a signal (50) comprising an identifier of a potential ictal of the subject;
   a data acquisition unit (120) adapted to obtain a current interictal period of the subject;
   a data processing unit (122) configured to generate an ictal detection threshold based on the current interictal period of the subject;
   an ictal detection component (124) adapted to detect an ictal of the subject based on the identifier of a potential ictal of the subject and the ictal detection threshold; and
   an output interface (160) configured to output information indicative of the detected ictal of the subject.

2. The system of claim 1, wherein the data acquisition unit (120) is adapted to obtain a current interictal period of the subject based on at least one of: a control signal (60) from a user or control system, and an output signal from the ictal detection component identifying the timing of a detected ictal of the subject.

3. The system of claim 1 or 2, wherein the data processing unit (122) is adapted to process the current interictal period in accordance with an interictal model to determine the ictal detection threshold, the interictal model being representative of a relationship between interictal period and a probability of the subject being in an interictal state at a point in time.

4. The system of claim 3, wherein the interictal model defines, for each of a plurality of values of duration of current interictal period, posterior odds of the subject being in the interictal state.

5. The system of any of claims 1 to 4, wherein the data processing unit (122) is adapted to determine a value for the ictal detection threshold such that the posterior odds of the subject being in an interictal state given the current interictal period meet a predetermined requirement.

6. The system of claim 5, wherein the predetermined requirement comprises: the posterior odds remain constant or the posterior odds decrease with elapsed time.

7. The system of any of claims 1 to 6, further comprising:

   a signal generator (55) adapted to generate a signal (50) comprising an identifier of a potential ictal of the subject based on at least one of: physiological data relating to one or more physiological properties of the subject, and activity data relating to activity or movement of the subject.

8. The system of claim 7, further comprising a first sensor (70) adapted to detect a value of activity or movement of the subject and to generate a signal comprising activity data representative of the detected value of activity or movement,
   and preferably wherein the first sensor comprises at least one of: an accelerometer; a gyroscope; a movement sensor; a weight sensor; a pressure sensor; and a timing device.

9. The system of claim 7 or 8, further comprising a second sensor (75) adapted to detect a value of a physiological property of the subject and to generate a signal comprising physiological data representative of the detected value a physiological property,
   and preferably wherein the second sensor comprises at least one of: a blood glucose sensor; a blood pressure sensor; a pulse rate sensor; an electrocardiograph sensor; a respiration sensor; and a blood oxygen saturation sensor.

10. A method for detecting an ictal of a subject, the method comprising:

obtaining (270) a signal comprising an identifier of a potential ictal of the subject;

obtaining (220) a current interictal period (230) of the subject;

generating (240) an ictal detection threshold (250) based on the current interictal period of the subject;

detecting (290) an ictal of the subject based on the identifier of a potential ictal of the subject and the ictal detection threshold; and

outputting (300) information indicative of the detected ictal of the subject.

11. The method of claim 10, wherein obtaining the current interictal period (230) of the subject is based on at least one of: a control signal from a user or control system, and an output signal from the ictal detection component identifying the timing of a detected ictal of the subject.

12. The method of claim 10 or 11, wherein generating (240) an ictal detection threshold comprises processing the current interictal period in accordance with an interictal model to determine the ictal detection threshold, the interictal model being representative of a relationship between interictal period and a probability of the subject being in an interictal state at a point in time.

13. The method of any of claims 10 to 12, wherein generating (240) an ictal detection threshold comprises determining a value for an ictal detection threshold such that the posterior odds of the subject being in an interictal state given the current interictal period meet a predetermined requirement,

and preferably wherein the predetermined requirement comprises: the posterior odds remain constant; or the posterior odds decrease with elapsed time.

14. The method of any of claims 10 to 13, further comprising generating a signal comprising an identifier of a potential ictal of the subject based on at least one of:

physiological data relating to one or more physiological properties of the subject, and activity data relating to activity or movement of the subject

15. A computer program product comprising computer readable code storable on, or stored on, or downloadable from a communications network, which code when run on a computer implements the method of any of claims 10 to 14.

FIG. 1

FIG. 2

EP 3 644 324 A1

FIG. 3

FIG. 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | P. SUFFCZYNSKI ET AL: "Dynamics of Epileptic Phenomena Determined From Statistics of Ictal Transitions", IEEE TRANSACTIONS ON BIOMEDICAL ENGINEERING., vol. 53, no. 3, 1 March 2006 (2006-03-01), pages 524-532, XP055575171, PISCATAWAY, NJ, USA. ISSN: 0018-9294, DOI: 10.1109/TBME.2005.869800 * abstract * * page 526, column 1, paragraph 3 * * page 529, column 2, paragraph 2 * ----- | 1-15 | INV. G16H50/20 ADD. G16H50/50 |
| A | US 2017/196497 A1 (RAY LAURA [US] ET AL) 13 July 2017 (2017-07-13) * abstract * ----- | 1-15 | |
| A | US 2013/261490 A1 (TRUCCOLO WILSON [US] ET AL) 3 October 2013 (2013-10-03) * abstract * * paragraph [0007]; claim 1 * ----- | 1-15 | |

TECHNICAL FIELDS
SEARCHED     (IPC)

G16H

The present search report has been drawn up for all claims

| Place of search | Date of completion of the search | Examiner |
|---|---|---|
| Munich | 1 April 2019 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 20 1673

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2019

| Patent document cited in search report | | Publication date | Patent family member(s) | | Publication date |
|---|---|---|---|---|---|
| US 2017196497 | A1 | 13-07-2017 | NONE | | |
| US 2013261490 | A1 | 03-10-2013 | US 2013261490 | A1 | 03-10-2013 |
| | | | WO 2012078503 | A2 | 14-06-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SUFFCZYNSKI et al.** Dynamics of Epileptic Phenomena Determined From Statistics of Ictal Transitions. *IEEE TRANSACTIONS ON BIOMEDICAL ENGINEERING,* March 2006, vol. 53 (3), 524-532